# EUROPÄISCHE PATENTSCHRIFT

(11) **EP 1 406 534 B1**
(45) Veröffentlichungstag und Bekanntmachung des Hinweises auf die Patenterteilung: **10.12.2008**
(21) Anmeldenummer: 02758325.1
(22) Anmeldetag: 08.07.2002
(51) Int. Cl.: A61B 3/113

(54) **VERFAHREN UND VORRICHTUNG ZUR VERFOLGUNG VON AUGENBEWEGUNGEN**
METHOD AND DEVICE FOR TRACKING EYE MOVEMENTS
PROCEDE ET DISPOSITIF DE SUIVI DE MOUVEMENTS OCULAIRES

(30) Priorität: 06.07.2001 DE 10132378
(43) Veröffentlichungstag der Anmeldung: 14.04.2004
(73) Patentinhaber: Carl Zeiss Meditec AG, 07745 Jena (DE)
(72) Erfinder: DICK, Manfred, 07926 Gefell (DE); FIEDLER, Joachim, 74564 Crailsheim (DE)
(74) Vertreter: DTS München
(86) Internationale Anmeldenummer: PCT/EP2002/007591
(87) Internationale Veröffentlichungsnummer: WO 2003/003909

(56) Entgegenhaltungen:
- EP-A- 0 850 614
- WO-A-00/27273
- WO-A-99/65381
- US-A- 5 980 513
- TELFAIR W B ET AL: "SCANNING MID-IR LASER APPARATUS WITH EYE TRACKING FOR REFRACTIVE SURGERY" PROCEEDINGS OF THE SPIE, SPIE, BELLINGHAM, VA, US, Bd. 3591, 23. Januar 1999 (1999-01-23), Seiten 220-228, XP008001611

## Beschreibung

Die vorliegende Erfindung betrifft ein Verfahren und eine Vorrichtung zur Verfolgung von Augenbewegungen.

Die refraktive Laserchirurgie der Cornea insbesondere mit ablativ arbeitenden Excimerlasern (ArF - Wellenlänge 193 nm) als OP-Laser ist mittlerweile zu einem etablierten Verfahren zur Behandlung von refraktiven Sehfehlern geworden. Die Behandlung wird dabei mit sogenannten Spotscanning-Systemen am frei beweglichen Auge des Patienten durchgeführt. Diese Systeme lassen auch patientenindividuelle Korrekturen, sogenannte customized ablations zu.

Eine Augenbewegung des Patienten macht es erforderlich, den Strahl des OP-Lasers entsprechend der Bewegung nachzuführen. Dazu wird mit einem sogenannten Eye-Tracking-System die Augenbewegung des Patienten und damit die aktuelle Stellung des Auges erfasst und als Korrekturwert zur Steuerung des OP-Lasers benutzt. Übliche Eye-Tracking-System arbeiten auf Basis bildverarbeitender Systeme, in dem diese beispielsweise die Pupille des Auges mit Hilfe einer Digitalkamera vermessen und mit Bildverarbeitungsalgorithmen durch Vergleich von Bildfolgen die Augenstellung bestimmen können. Da Augenbewegungen recht schnell erfolgen und bereits kleinste Winkelfehler erkannt werden müssen, werden dazu üblicherweise Hochgeschwindigkeits-Video-Kameras eingesetzt.

Als Tracking-Objekt für die Hochgeschwindigkeits-Video-Kamera verwendet man vorzugsweise die Pupille, da man dadurch am Anfang eine automatische videogestützte Pupillenzentrierung durchführen kann und das gleiche Objekt auch während der Behandlung nachverfolgen kann. Diese Vorgehensweise ist am unbehandelten Auge problemlos. Bereits bei vorbereitenden Arbeiten wie dem Entfernen des Epithels bei der PRK (Photo-Refraktive Keratektomie) oder dem Aufklappen des Flaps bei LASIK (Laser assisted in situ Keratumilensis) verändert man jedoch die Kontrastverhältnisse. Während der Laserbehandlung wird dieses Problem weiter verschärft, da man eine stark streuende Oberfäche über den Pupillendurchmesser hinaus erzeugt.

Um dieses Problem zu umgehen, wurden verschiedene Optimierungsansätze erarbeitet. Dabei weicht man auf andere Tracking-Objekte am Auge wie z. B. Limbus oder Iris nach anfänglicher Pupillenzentrierung aus oder legt zusätzliche Trackingringe auf das Auge, um eine stabile Funktion des Eye Trackings während der Behandlung zu gewährleisten. Dies ist jedoch mit unangenehmem zusätzlichem Behandlungsaufwand verbunden.

Bei einem anderen Optimierungsansatz wird das Auge mit Infrarot-LEDs seitlich beleuchtet. Dadurch erhält man auch während der Laserbehandlung eine geringere Ausfallrate des Systems.

Die Beleuchtung ist jedoch mit zusätzlichem Arbeitsaufwand für den Arzt und einer Einschränkung der Arbeitsfreiheit im OP-Feld verbunden.

In der EP-A-0850614 wird eine ophtalmologische Operationsvorrichtung beschrieben, entsprechend dem Oberbegriff des Anspruchs 1 und des Anspruchs 18.

In der WO 00/27273 wird ein "Eye Tracking System" für die refraktive Operation beschrieben, bei der mehrere für das Auge unbedenklichen Lichtstrahlen auf das Auge gerichtet werden und einen Bereich auf der Augenoberfläche bestrahlen, der größer ist als die Pupille, und ebenfalls entsprechend dem Oberbegriff des Ansprüche 1 und 18.

In der WO 99/65381 wird ein "Eye Tracking System" beschrieben, durch das die Blickrichtung eines Auges verfolgt werden soll.

Der vorliegenden Erfindung liegt daher das Problem zugrunde, ein Verfahren und eine Vorrichtung zur Verfolgung von Augenbewegungen bereitzustellen, die eine zuverlässige Verfolgung der Augenbewegung bei möglichst einfacher Bedienung gewährleisten.

Dieses Problem wird durch ein Verfahren nach Patentanspruch 1 sowie eine Vorrichtung nach Patentanspruch 18 gelöst.

Das erfindungsgemäße Verfahren sieht vor, dass die Bestrahlung durch die Pupille erfolgt bzw. die Pupille durch das reflektierte Licht eine quasi monochromatische IR-Quelle bildet.

In einer Weiterbildung des Verfahrens ist vorgesehen, dass die Anregung des Augeninneren zum Leuchten durch eine koaxial zur optischen Achse eines OP-Lasers angeordnete Lichtquelle erfolgt. Dieses Verfahren bietet den Vorteil, dass der behandelnde Arzt nur eine einzige Strahlungsquelle handhaben muss.

In einer Weiterbildung des Verfahrens ist vorgesehen, dass die Lichtquelle eine IR-Laserdiode ist. Auf diese Weise lässt sich eine ausreichende Leuchtintensität ohne störende Lichtreflexe beispielsweise für den behandelnden Arzt erzielen.

In einer Weiterbildung des Verfahrens ist vorgesehen, dass die IR-Laserdiode gepulst arbeitet. Dies ermöglicht eine hohe augenblickliche Strahlungsleistung mit entsprechend hoher Leuchtdichte des rückgestrahlten Lichtes bei einer vergleichsweie geringen mittleren Bestrahlungsleistung.

In einer Weiterbildung des Verfahrens ist vorgesehen, dass die Impulse der IR-Laserdiode mit der Laserschussfrequenz des OP-Lasers so synchronisiert sind, dass die IR-Laserdiode nur in Laserschusspausen des OP-Lasers leuchtet. Diese Maßnahme verringert eventuelle Interfrerenzen oder Beeinträchtigung der Kameraoptik durch den verwendeten OP-Laser.

In einer Weiterbildung des Verfahrens ist vorgesehen, dass die Impulse der IR-Laserdiode mit der Bildfrequenz einer Kamera, die das von dem Auge emittierte Licht aufnimmt, synchronisiert sind. Die Kamera nimmt daher nur Bilder auf, die während der Bestrahlung des Auges durch die IR-Laserdiode erzeugt werden. Diese Maßnahme vermindert Ungenauigkeiten durch von dem OP-Laser erzeugte Bilder weiter.

In einer Weiterbildung des Verfahrens ist vorgesehen, dass die Strahlung der Laserdiode einen Fokus auf der Corneaoberfläche des Auges hat. Auf diese Weise wird das Auge selbst in seinen optischen Eigenschaften optimal ausgenutzt.

In einer Weiterbildung des Verfahrens ist vorgesehen, dass die Strahlung der Laserdiode einen möglichst ausgedehnten Fleck auf der Netzhaut des Auges hat. Mit dieser Maßnahme wird die Intensität des rückgestreuten Lichtes optimiert.

Das eingangs genannte Problem wird auch durch eine Vorrichtung zur Verfolgung von Augenbewegungen insbesondere bei der refraktiven Laserchirurgie umfassend eine Kamera, eine Auswerteeinheit sowie eine Lichtquelle gelöst, wobei diese nach einem Verfahren nach einem der Ansprüche 1 bis 9 arbeiten kann. In einer bevorzugten Ausführungsform ist dabei vorgesehen, dass die Strahlung der Lichtquelle und das vom Auge rückgestrahlte Licht im Wesentlichen koaxial verlaufen. Auf diese Weise lassen sie sämtliche bei der refraktiven Laserchirurgie des Auges zu bedienende Elemente in einem einzigen Gehäuse und dabei nur einer von dem Arzt zu bedienenden Vorrichtung zusammenfassen.

In einer Weiterbildung der Vorrichtung ist vorgesehen, dass die Strahlung der Lichtquelle und das vom Auge rückgestrahlte Licht im Wesentlichen koaxial verlaufen. Diese Maßnahme ermöglicht eine weitere Zusammenfassung der Baugruppen in nur einer Gehäuseeinheit.

In einer Weiterbildung der Vorrichtung ist vorgesehen, dass das rückgestrahlte Licht durch einen Scannerspiegel auf die nicht koaxial zur Strahlung der Beleuchtungseinheit angeordnete Kamera gelenkt wird. Mit Hilfe des Scannerspiegels kann so weit entfernt vom Auge eine Aufteilung beider Strahlengänge erfolgen.

Der Scannerspiegel ist vorzugsweise so gestaltet, dass dieser den überwiegenden Teil des von der Lichtquelle auf diesen auftreffenden Lichtes durchlässt und damit das Auge bestrahlt wird und das vom Auge reflektierte Licht reflekiert. Spiegel lassen sich auch mit dielektrischer Beschichtung nur für Transmission oder Reflexion optimieren. Durch die geringere von dem Auge rückgestrahlte Lichtintensität und die Möglichkeit, die Leistung der Lichtquelle nahezu beliebig zu wählen wird der Scannerspiegel im vorliegenden Anwendungsfall für die Reflexion optimiert.

In einer Weiterbildung der Vorrichtung ist vorgesehen, dass im Strahlengang des reflektierten Lichtes ein Filter angeordnet ist, der im wesentlichen Licht der von der Lichtquelle abgegebenen Wellenlänge durchlässt. Störstrahlung, die aus der Umgebung auf das Auge trifft und von diesem reflektiert wird, kann so herausgefiltert werden. Die Intensität des von der Lichtquelle auf das Auge eingestrahlten Lichtes kann mit Rücksicht auf die Belastbarkeit der Retina des Auges nicht beliebig erhöht werden. Eine Verbesserung des Störstrahlungsabstandes wird daher hier bewirkt durch das Herausfiltern des Störspektrums. Diese Maßnahme erhöht das Kontrastverhältnis zwischen Pupille und Umgebungsbereich.

Bevorzugt wird ein dielektrisches Filterelement eingesetzt, mit dem die Umgebungsintensität aufgrund der Monochromatie der Laserstrahlung stark unterdrückt wird und so ein gutes Kontrastverhältnis erzeugt werden kann.

In einem weiteren bevorzugten Ausführungsbeispiel der vorliegenden Erfindung wird zur Bestimmung der Augenposition ein Fixpunkt auf der Cornea bestimmt. Ein solcher Fixpunkt dient dazu, die Augenbewegung nachverfolgen zu können und in Referenz zu diesem Punkt einen "Off-set" für die aktuellen Koordinaten des Auges zu ermitteln. In Abweichung von diesem Fixpunkt können dann beispielsweise die Schußparameter und koordinaten ermittelt werden.

Besonders bevorzugt ist dieser Fixpunkt der geometrische Mittelpunkt des optischen Abbildes der Pupille. Dieser geometrische Mittelpunkt bzw. Schwerpunkt der Pupille wird dann als Fixpunkt übernommen. Da sich die Pupille im Durchmesser verändert, verschiebt sich dieser Mittelpunkt über die Zeit. Daher wird dieser Mittelpunkt bzw. Fixpunkt bevorzugt regelmäßig neu bestimmt. Damit wird der Fixpunkt auf der Cornea gehalten. Von der Zuordnung dieses Fixpunktes wird dann der "Off-set" bestimmt, der beispielsweise auf einen Scannerspiegel gegeben werden kann.

In einem weiteren bevorzugten Ausführungsbeispiel der vorliegenden Erfindung wird durch die Auswertung des Abbildes der Pupille die laterale Verschiebung des Auges und/oder die Rollbewegung des Auges erfasst. Hierdurch können kugelförmige Objekte, wie beispielsweise das Auge, mit Lasern behandelt werden, wobei es nun berücksichtigt werden kann, ob der Ablations-Spot des Lasers auf den Mittelpunkt der Kugel bzw. des Auges gerichtet ist oder der Laserschuß in seiner gedachten Verlängerung weit am Mittelpunkt des Auges bzw. der Kugel vorbeiführt. Davon ist nämlich der Wirkungsgrad des Ablations-Spots des Lasers abhängig. Wenn dieser die Kugeloberfläche peripher am Rand trifft, d.h. der gedachte verlängerte Laserschuß am Mittelpunkt des Auges vorbeigeht, dann muß für dieselbe Ablationswirkung eine höhere Energie aufgewendet werden, da der Laser-Spot nun als elliptische Projektion des kreisförmigen Spots auf der Kugel- bzw. Augenoberfläche auftrifft. Wenn der Schuß genau auf den Mittelpunkt der Kugel bzw. des Auges gezielt ist, dann ist der Wirkungsgrad des Laser-Spots in der Ablation am höchsten. Durch die Berücksichtigung der Rollbewegung des Auges neben der translatorischen bzw. lateralen Verschiebung können diese Energieeffekt ausgeglichen werden und jeweils unter Berücksichtigung des optimalen Wirkungsgrades genau an Hand der Position des Auges und des anzubrungenden Ablations-Spots berücksichtigt werden. Damit ist eine och genauere Bearbeitung der Oberfläche möglcih.Das Abrollen bzw. Rollen des Auges kann durch Bildverarbeitung ermittelt werden, insbesondere über die Auswertung der erfaßten Irisstruktur. Bevorzugt wird dabei auch die Vorderkammertiefe des Auges berücksichtigt. Damit läßt sich der Drehwinkel des Auges dann ermitteln und somit auch dieser Effekt bezüglich des Energieeintrages auf der Kugeloberfläche außerhalb des Mittelpunktes berücksichtigen.

In einem weiteren bevorzugten Verfahren der vorliegenden Erfindung wird aus einer mittleren Augenlänge und einer mittleren Voderkammertiefe ein Ausgleichsfaktor berechnet, welcher der Parallaxe zwischen Hornhautvorderfläche und Eintrittspupille entspricht. Dadurch können die Abweichungen, die sich durch die Rollbewegung des Auges ergeben kann, kompensiert werden. Bevorzugt wird hierbei ein mittlererWert für die biometrischen Parameter wie Augenlänge und Vorderkammertiefe verwendet. Somit lassen sich aufwendige Vorabmessungen vermeiden, was zu einer zügigeren Duchführung des Verfahrens führt.

Bevorzugt wird der Ausgleichsfaktor auf Scannerspiegel eines Arbeitslasers übertragen. Damit können die gewonnenen Werte zur Kompensation der Parallaxe direkt auf die Scannerspiegel weitergegeben werden, die einen Arbeitslaserstrahl ablenken und so dafür sorgen, dass die Parallaxe direkt auf der zu bearbeitenden Kugeloberfläche kompensiert werden kann.

Bevorzugt ist der Ausgleichsfaktor linear. Für kleine Winkel in dieser geometrisch bestimmten Parallaxe gilt beispielsweise die Näherung, dass der Sinus durch das Argument ersetzt werden kann. Daneben existieren weitere Näherungsmöglichkeiten, die eine einfache Bestimmung dieses Ausgleichsfaktors ermöglichen. Dieser lineare (genäherte) Ausgleichsfaktor ist einfach und schnell zu bestimmen und erlaubt so eine zügige Durchführung des erfindungsgemäßen Verfahrens.

Besonders bevorzugt gehorcht der Ausgleichsfaktor einer nichtlinearen Funktion. Diese nichtlineare Funktion zur Ermittlung des Ausgleichsfaktors ist die geometrisch korrekte Funktion, während die lineare Beziehung eine Näherung darstellt. Gerade für größere Winkel in der Parallaxe können die Näherungen zu unerwünschten Abweichungen bzw. Fehlern führen. Mit dem bevorzugten Verfahren können damit größere Abweichungen besser kompensiert werden.

Bevorzugt wird der Ausgleichsfaktor durch die individuelle Messung biometrischer Parameter bestimmt. Solche biometrischen Parameter sind insbesondere die Augenlänge und/oder die Vorderkammertiefe. Besonders bevorzugt wird die Augenlänge individuell bestimmt, da diese einfach zu messen ist. Ganz besonders bevorzugt wird (auch) die Vorderkammertiefe individuell bestimmt, da hiermit eine beonders korrekte Kompensation individuell durchgeführt werden kann.

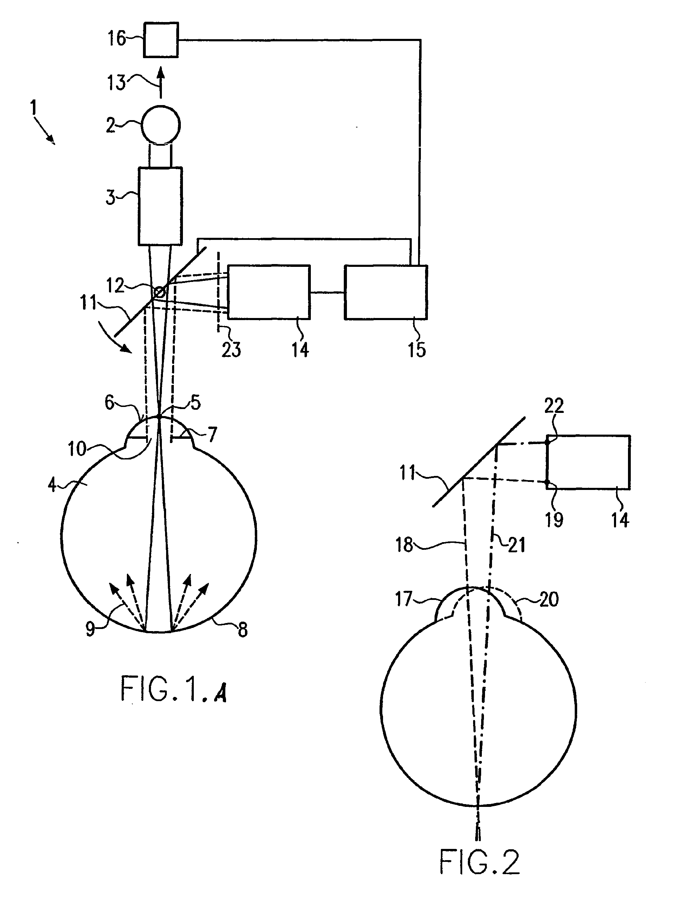

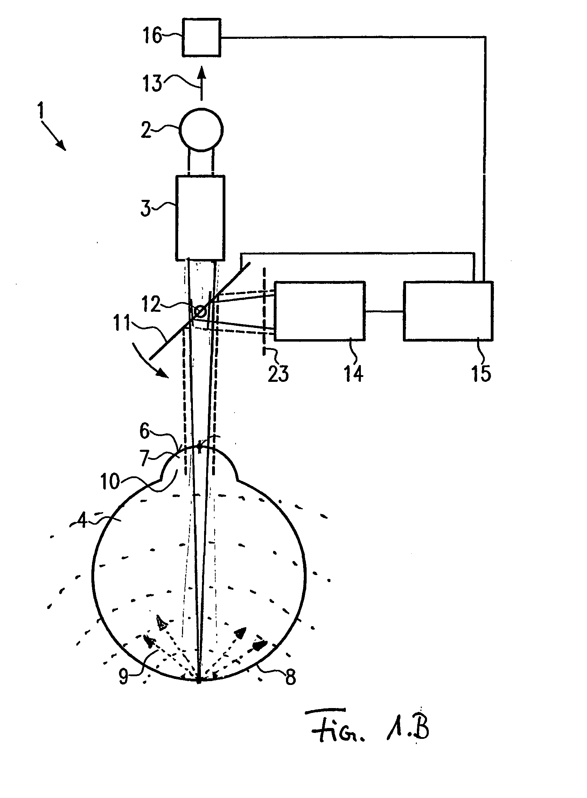

Weiter vorteilhafte Ausgestaltungen der Erfindung werden nachfolgend anhand der Zeichnungen erläutert. Dabei zeigen
- Fig. 1 a: eine Prinzipskizze des Aufbaus der Vorrichtung;
- Fig. 1 b: eine Prinzipskizze des Aufbaus eines weitren Ausführungsbeispiels der erfindungsgemäßen Vorrichtung;
- Fig. 2: eine Prinzipskizze des Strahlenganges der Vorrichtung.

Eine Vorrichtung zur Verfolgung der Augenbewegung 1 umfasst eine Lichtquelle 2, sowie ein Teleskop 3. Das von der Lichtquelle 2 ausgesandte Licht wird über das Teleskop 3, das mit weiteren optischen Einrichtungen wie beispielsweise Linsen oder dergleichen ausgestattet sein kann, auf ein Auge 4 einer nicht dargestellten zu behandelnden Person gelenkt. Das Licht wird in einem Fokus 5 gebündelt. Der Fokus 5 liegt auf der Corneaoberfläche 6 der Iris 7 des Auges 4. Nach dem Durchtritt durch die Corneaoberfläche 6 weitet sich der Lichtstrahl der Lichtquelle 2 auf und trifft als ausgedehnter Fleck auf die Netzhaut 8 des Auges 4. Die Lichtquelle 2 ist im vorliegenden Ausführungsbeispiel eine Infrarot-Laserdiode. Deren Leistung ist so gewählt, dass die zulässige Bestrahlungsintensität für die Netzhaut 8 eingehalten wird.

Von der Netzhaut 8 wird das darauf treffende Licht diffus zurückgestrahlt, dies ist durch Pfeile 9 in Figur 1 angedeutet. Das von der Netzhaut 8 zurückgestrahlte Licht 9 führt zu einer homogenen Ausleuchtung der Pupille 10.

Nach Durchtritt des zurückgestrahlten Lichtes 9 durch die Pupille 10 trifft dieses auf einen Scannerspiegel 11. Dieser ist um mindestens zwei Achsen beweglich, beispielsweise um die aus der Zeichenebene herausragende Querachse 12 sowie die in Figur 1 mit dem Bezugszeichen 13 bezeichnete Hochachse. Durch Bewegung um diese beiden Achsen kann der Scannerspiegel 11 unterschiedliche Bereiche der Netzhaut 8 des Auges 4 auf eine Kamera 14 abbilden. Die Kamera 14 ist an einer Auswerteeinheit 15 angeschlossen, die zum Einen den Scannerspiegel 11 und zum Anderen einen nicht näher dargestellten OP-Laser 16 steuert. Die optische Achse des OP-Lasers 16 ist koaxial zu der optischen Achse der Vorrichtung zur Augenverfolgung 1 angeordnet.

Der Scannerspiegel 11 ist so gestaltet, dass dieser nur einen geringen Teil des Lichtes, beispielsweise etwa 2% durchlässt und den größten Teil des Lichts, beispielsweise etwa 98%, reflektiert. Alternativ kann der Scannerspiegel 11 mit einem kleinen Loch versehen sein, oder die Kamera 14 kann das Auge 4 im nicht koaxialen Strahlengang beobachten.

Die Infrarot-Laserdiode der Lichtquelle 2 wird gepulst betrieben. Die Lichtimpulse werden mit Hilfe der Auswerteeinheit 15 synchronisiert mit der Laserschussfrequenz des OP-Lasers 16. Die so entstehende gepulste Beleuchtung durch die IR-Laserdiode minimiert die Belastung der Netzhaut 8 und erhöht den Kontrast zwischen beleuchteten und nicht beleuchteten Bereichen.

Die Infrarot-Laserdiode der Lichtquelle 2 gibt ein monochromatisches Licht ab. Das vom Auge reflektierte Licht hat die gleiche Wellenlänge wie das von der Infrarot-Laserdiode abgegebene Licht. Diese Wellenlänge wird beispielsweise durch einen dielektrischen Filter herausgefiltert. Dadurch werden andere Wellenlängen, die beispielsweise aus der Umgebung auf das Auge strahlen und von diesem reflektiert werden oder direkt auf die Kamera treffen, herausgefiltert. Dadurch kann der Kontrast zwischen beleuchteten und nicht beleuchteten Bereichen bzw. der Pupille und deren Umgebung weiter erhöht werden.

In **Figur 1** **B** ist ein weiteres nicht zur Erfindung gehörendes Beispiel dargestellt. Der Aufbau entspricht dem der Vorrichtung aus Figur 1 A. Hier wird der Fokus des Strahles nicht auf die Pupille sondern direkt auf die Retina gerichtet. Der Fokus der IR-Quelle auf der Retina erzeugt nun Floureszenz. Durch Kugelwellen (gestrichelt eingezeichnet), die jetzt von der Retina zurückgeworfen werden, wird eine homogene Ausleuchtung der Pupille erreicht. Dies führt zu einer bevorzugten regredienten Beleuchtung. Somit kann die Retina selbst als Lichtquelle und nicht als Lichtfalle fungieren.

**Figur 2** verdeutlicht die Bestimmung der Augenbewegung bzw. der Augenstellung an zwei Beispielen der Augenstellung. In einer mit durchgezogener Linie der Iris 7 dargestellten ersten Augenstellung 17 wird ein erster Lichtstrahl 18 über den Scannerspiegel 12 auf einen ersten Abbildungspunkt 19 der Kamera 14 gelenkt. Durch Bewegung des Scannerspiegels 11 um seine Querachse 12 sowie die Hochachse 13 kann so ein vollständiges Abbild eines Bereiches der Netzhaut 8 auf der Kamera 14 erfolgen.

Bei Veränderung der Augenstellung in eine zweite Augenstellung 20 erzeugt der gleiche Punkt der Netzhaut 8 nunmehr einen zweiten Lichtstrahl 21, der in einem zweiten Abbildungspunkt 22 auf die Kamera 14 trifft. Bei einer Bewegung des Scannerspiegels 11 entsprechend der vorherigen Darstellung wird ebenfalls eine Abbildung eines Bereiches der Netzhaut 8 auf der Kamera 14 erzielt. Diese Abbildung der Netzhaut 8 ist gegenüber der ersten Augenstellung 17 auf der Kamera 14 verschoben. Durch einen geeigneten rechnerischen Vergleich der digitalisierten Bilder der Kamera 14 mit Hilfe der Auswerteeinheit 15 kann so auf die veränderte Winkelstellung des Auges 4 zurückgeschlossen werden. Der an der Corneaoberfläche 6 entstehende punktuelle Reflex bzw. Streupunkt wird von dem bildbearbeitenden Videosystem der Auswerteeinheit 15 ausgeblendet.

### BEZUGSZEICHENLISTE

- 1: Vorrichtung zur Verfolgung der Augenbewegung
- 2: Lichtquelle
- 3: Teleskop
- 4: Auge
- 5: Fokus
- 6: Corneaoberfläche
- 7: Iris
- 8: Netzhaut
- 9: Rückgestrahltes Licht
- 10: Pupille
- 11: Scannerspiegel
- 12: Querachse
- 13: Hochachse
- 14: Kamera
- 15: Auswerteeinheit
- 16: OP-Laser
- 17: Erste Augenstellung
- 18: Erster Lichtstrahl
- 19: Erster Abbildungspunkt
- 20: Zweite Augenstellung
- 21: Zweiter Lichtstrahl
- 22: Zweiter Abbildungspunkt
- 23: Filter

## Patentansprüche

1. Verfahren zur Verfolgung von Augenbewegungen eines Auges (4) mit einer Netzhaut (8) bei der refraktiven Laserchirurgie, wobei das Augeninnere durch Bestrahlung mit einem Strahl vom Augenäusseren her zum Leuchten angeregt wird und das aus der Pupille (10) des Auges (4) austretende Licht zur Bestimmung der Augenposition benutzt wird, wobei die Bestrahlung durch die Pupille (10) erfolgt
**dadurch gekennzeichnet, dass**
der Fokus des Strahles auf die Cornea des Auges (4) gerichtet ist.

2. Verfahren nach einem der vorhergehenden Ansprüche,
**dadurch gekennzeichnet, dass**
die Anregung des Augeninneren zum Leuchten durch eine koaxial zur optischen Achse eines OP-Lasers (16) angeordnete Lichtquelle (2) erfolgt.

3. Verfahren nach Anspruch 2,
**dadurch gekennzeichnet, dass**
die Lichtquelle (2) eine IR-Laserdiode ist.

4. Verfahren nach einem der Ansprüche 2 oder 3 ,
**dadurch gekennzeichnet, dass**
die IR-Laserdiode gepulst arbeitet.

5. Verfahren nach Anspruch 4,
**dadurch gekennzeichnet, dass**
die Impulse der IR-Laserdiode mit der Laserschussfrequenz des OP-Lasers (16) so synchronisiert sind, dass die IR-Laserdiode nur in Laserschusspausen des OP-Lasers (16) leuchtet.

6. Verfahren nach einem der Ansprüche 4 oder 5 ,
**dadurch gekennzeichnet, dass**
die Impulse der IR-Laserdiode mit der Bildfrequenz einer Kamera (14), die das von dem Auge (4) emittierte Licht aufnimmt, synchronisiert sind.

7. Verfahren nach einem der vorhergehenden Ansprüche,
**dadurch gekennzeichnet, dass**
die Strahlung der Laserdiode einen Fokus (5) auf der Corneaoberfläche (6) des Auges (4) hat.

8. Verfahren nach einem der vorhergehenden Ansprüche,
**dadurch gekennzeichnet, dass**
die Strahlung der Laserdiode einen möglichst ausgedehnten Fleck auf der Netzhaut (8) des Auges (4) hat.

9. Verfahren nach einem der vorhergehenden Ansprüche,
**dadurch gekennzeichnet, dass**
zur Bestimmung der Augenposition ein Fixpunkt auf der Cornea bestimmt wird.

10. Verfahren nach Anspruch 9,
**dadurch gekennzeichnet, dass**
der Fixpunkt der geometrische Mittelpunkt des optischen Abbildes der Pupille ist.

11. Verfahren nach einem der Ansprüche 9 bis 10,
**dadurch gekennzeichnet, dass**
der Fixpunkt regelmäßig neu bestimmt wird.

12. Verfahren nach einem der vorhergehenden Ansprüche,
**dadurch gekennzeichnet, dass**
durch die Auswertung des Abbildes der Pupille die laterale Verschiebung des Auges und/ oder die Rollbewegung des Auges erfasst wird.

13. Verfahren nach einem der vorhergehenden Ansprüche,
**dadurch gekennzeichnet, dass**
aus einer mittleren Augenlänge und einer mittleren Vorderkammertiefe ein Ausgleichsfaktor berechnet wird, welcher der Parallaxe zwischen Hornhautvorderfläche und Eintrittspupille entspricht.

14. Verfahren nach Anspruch 13,
**dadurch gekennzeichnet, dass**
der Ausgleichsfaktor auf Scannerspiegel eines Arbeitslasers übertragen wird.

15. Verfahren nach einem der Ansprüche 13 bis 14,
**dadurch gekennzeichnet, dass**
der Ausgleichsfaktor linear ist.

16. Verfahren nach einem der Ansprüche 13 bis 14,
**dadurch gekennzeichnet, dass**
der Ausgleichsfaktor einer nichtlinearen Funktion gehorcht.

17. Verfahren nach einem der Ansprüche 13 bis 16,
**dadurch gekennzeichnet, dass**
der Aüsgleichsfaktor durch individuelle Messung biometrischer Parameter bestimmt wird.

18. Vorrichtung (1) zur Verfolgung von Augenbewegungen eines Auges (4) mit einer Netzhaut (8) bei der refraktiven Laserchirurgie umfassend eine Kamera (14), eine Auswerteeinheit (15) zur Steuerung eines OP-Lasers (16) sowie eine Lichtquelle (2),
**dadurch gekennzeichnet, dass**
ein Fokus des aus der Lichtquelle (2) austretenden Strahles auf die Cornea des Auges (4) ausrichtbar ist.

19. Vorrichtung nach Anspruch 18,
**dadurch gekennzeichnet, dass**
die Strahlung der Lichtquelle (2), und das vom Auge (4) rückgestrahlte Licht im wesentlichen koaxial verlaufen.

20. Vorrichtung nach Anspruch 19,
**dadurch gekennzeichnet, dass**
das rückgestrahlte Licht durch einen Scannerspiegel (11) auf die nicht koaxial zur Strahlung der Lichtquelle (2) angeordnete Kamera (14) gelenkt wird.

21. Vorrichtung nach Anspruch 20,
**dadurch gekennzeichnet, dass**
der Scannerspiegel (11) den überwiegenden Teil des von der Lichtquelle (2) auf diesen auftreffenden Lichtes durchlässt und damit das Auge bestrahlt wird und das vom Auge rückgestrahlte Licht reflekiert.

22. Vorrichtung nach einem der Ansprüche 18 bis 21,
**dadurch gekennzeichnet, dass**
im Strahlengang des reflektierten Lichtes ein Filter (23) angeordnet ist, der im wesentlichen Licht der von der Lichtquelle (2) abgegebenen Wellenlänge durchlässt.

23. Vorrichtung nach Anspruch 22,
**dadurch gekennzeichnet, dass**
der Filter (23) ein dielektrisches Filterelement ist.

## Claims

1. Method for tracking eye movements of an eye (4) with a retina (8) during refractive laser surgery, the inside of the eye being excited to illumination by irradiation with a beam from the outside of the eye, and the light emerging from the pupil (10) of the eye (4) being used to determine the eye position, the irradiation taking place through the pupil (10),
**characterized in that**
the focus of the beam is directed onto the cornea of the eye (4).

2. Method according to one of the previous claims,
**characterized in that**
the excitation to illumination of the inside of the eye takes place through a light source (2) arranged coaxial to the optical axis of an OP laser (16).

3. Method according to claim 2,
**characterized in that**
the light source (2) is an IR laser diode.

4. Method according to one of claims 2 or 3,
**characterized in that**
the IR laser diode operates pulsewise.

5. Method according to claim 4,
**characterized in that**
the pulses of the IR laser diode are synchronized with the laser firing frequency of the OP laser (16) such that the IR laser diode illuminates only in laser firing pauses of the OP laser (16).

6. Method according to one of claims 4 or 5,
**characterized in that**
the pulses of the IR laser diode are synchronized with the image frequency of a camera (14) which absorbs the light emitted by the eye (4).

7. Method according to one of the previous claims,
**characterized in that**
the radiation of the laser diode has a focal point (5) on the surface of the cornea (6) of the eye (4).

8. Method according to one of the previous claims,
**characterized in that**
the radiation of the laser diode has a spot which is broadened as much as possible on the retina (8) of the eye (4).

9. Method according to one of the previous claims,
**characterized in that**
a point of fixation is determined on the cornea to determine the eye position.

10. Method according to claim 9,
**characterized in that**
the point of fixation is the geometric centre of the optical image of the pupil.

11. Method according to one of claims 9 to 10,
**characterized in that**
the point of fixation is regularly redetermined.

12. Method according to one of the previous claims,
**characterized in that**
the lateral shift of the eye and/or the rolling movement of the eye is detected by the evaluation of the image of the pupil.

13. Method according to one of the previous claims,
**characterized in that**
there is calculated from an average eye length and an average anterior chamber depth a compensation factor which corresponds to the parallax between the front surface of the cornea and entrance pupil.

14. Method according to claim 13,
**characterized in that**
the compensation factor is transmitted to scanner mirrors of a working laser.

15. Method according to one of claims 13 to 14,
**characterized in that**
the compensation factor is linear.

16. Method according to one of claims 13 to 14,
**characterized in that**
the compensation factor obeys a non-linear function.

17. Method according to one of claims 13 to 16,
**characterized in that**
the compensation factor is determined by individual measurement of biometric parameters.

18. Device (1) for tracking eye movements of an eye (4) with a retina (8) during refractive laser surgery, comprising a camera (14), an evaluation unit (15) for steering an OP laser (16) and a light source (2),
**characterized in that**
a focus of the beam emerging from the light source (2) can be directed onto the cornea of the eye (4).

19. Device according to claim 18,
**characterized in that**
the radiation of the light source (2) and the light reflected from the eye (4) run essentially coaxial.

20. Device according to claim 19,
**characterized in that**
the reflected light is guided by a scanner mirror (11) onto the camera (14) which is arranged non-coaxial relative to the radiation of the light source (2).

21. Device according to claim 20,
**characterized in that**
the scanner mirror (11) passes most of the light striking it from the light source (2), and the eye is thus irradiated and the light reflected by the eye is reflected.

22. Device according to one of claims 18 to 21,
**characterized in that**
here is arranged in the beam path of the reflected light a filter (23) which essentially admits light of the wavelength emitted by the light source (2).

23. Device according to claim 22,
**characterized in that**
the filter (23) is a dielectric filter element.

## Revendications

1. Procédé de suivi de mouvements oculaires d'un oeil (4) doté d'une rétine (8) dans le domaine de la chirurgie laser réfractive, l'intérieur de l'oeil étant excité par rayonnement avec un faisceau depuis l'extérieur de l'oeil en vue de l'éclairer et la lumière sortant de la pupille (10) de l'oeil (4) étant utilisée pour déterminer la position de l'oeil, le rayonnement étant réalisé au travers de la pupille (10), **caractérisé en ce que** le foyer du faisceau est dirigé sur la cornée de l'oeil (4).

2. Procédé selon l'une quelconque des revendications précédentes, **caractérisé en ce que** l'excitation de l'intérieur de l'oeil en vue de l'éclairer est réalisée par le biais d'une source lumineuse (2) disposée de façon coaxiale par rapport à l'axe optique d'un laser OP (16).

3. Procédé selon la revendication 2, **caractérisé en ce que** la source lumineuse (2) est une diode laser à IR.

4. Procédé selon l'une quelconque des revendications 2 ou 3, **caractérisé en ce que** la diode laser à IR fonctionne de façon pulsée.

5. Procédé selon la revendication 4, **caractérisé en ce que** les impulsions de la diode laser à IR sont synchronisées de telle sorte avec la fréquence de décharge du laser OP (16) que la diode laser à IR n'éclaire que pendant les pauses de décharge du laser OP (16).

6. Procédé selon l'une quelconque des revendications 4 ou 5, **caractérisé en ce que** les impulsions de la diode laser à IR sont synchronisées avec la fréquence d'images d'une caméra (14) qui filme la lumière émise par l'oeil (4).

7. Procédé selon l'une quelconque des revendications précédentes, **caractérisé en ce que** le rayonnement de la diode laser a un foyer (5) situé sur la surface de la cornée (6) de l'oeil (4).

8. Procédé selon l'une quelconque des revendications précédentes, **caractérisé en ce que** le rayonnement de la diode laser a une tache la plus étendue possible sur la rétine (8) de l'oeil (4).

9. Procédé selon l'une quelconque des revendications précédentes, **caractérisé en ce qu'**un point fixe est déterminé pour déterminer la position de l'oeil sur la cornée.

10. Procédé selon la revendication 9, **caractérisé en ce que** le point fixe est le centre géométrique de l'image optique de la pupille.

11. Procédé selon l'une quelconque des revendications 9 à 10, **caractérisé en ce que** le point fixe est redéterminé régulièrement.

12. Procédé selon l'une quelconque des revendications précédentes, **caractérisé en ce que** l'analyse de l'image de la pupille permet de saisir le déplacement latéral de l'oeil et/ou le mouvement de roulement de l'oeil.

13. Procédé selon l'une quelconque des revendications précédentes, **caractérisé en ce qu'**un facteur de compensation est calculé à partir d'une longueur d'oeil moyenne et d'une profondeur de chambre antérieure moyenne, ledit facteur correspondant au parallaxe entre la surface avant de la cornée et la pupille d'entrée.

14. Procédé selon la revendication 13, **caractérisé en ce que** le facteur de compensation est transmis au miroir de balayage d'un laser de travail.

15. Procédé selon l'une quelconque des revendications 13 à 14, **caractérisé en ce que** le facteur de compensation est linéaire.

16. Procédé selon l'une quelconque des revendications 13 à 14, **caractérisé en ce que** le facteur de compensation obéit à une fonction non linéaire.

17. Procédé selon l'une quelconque des revendications 13 à 16, **caractérisé en ce que** le facteur de compensation est déterminé par mesure individuelle de paramètres biométriques.

18. Dispositif (1) de suivi des mouvements d'oeil d'un oeil (4) doté d'une rétine (8) dans le domaine de la chirurgie laser réfractive comprenant une caméra (14), une unité d'analyse (15) pour commander un laser OP (16) ainsi qu'une source lumineuse (2), **caractérisé en ce qu'**un foyer du faisceau sortant de la source lumineuse (2) peut être dirigé sur la cornée de l'oeil (4).

19. Dispositif selon la revendication 18, **caractérisé en ce que** le rayonnement de la source lumineuse (2) et la lumière réfléchie par l'oeil (4) évoluent pour l'essentiel de façon coaxiale.

20. Dispositif selon la revendication 19, **caractérisé en ce que** la lumière réfléchie est déviée sur la caméra (14) disposée de façon non coaxiale par rapport au rayonnement de la source lumineuse (2) par un miroir de balayage (11).

21. Dispositif selon la revendication 20, **caractérisé en ce que** le miroir de balayage (11) laisse passer la majeure partie de la lumière de la source lumineuse (2) incidente, exposant ainsi l'oeil aux rayons et réfléchissant la lumière réfléchie par l'oeil.

22. Dispositif selon l'une quelconque des revendications 18 à 21, **caractérisé en ce qu'**un filtre (23) est disposé dans le trajet du faisceau de la lumière réfléchie, ledit filtre laissant pour l'essentiel passer la lumière de la longueur d'onde émise par la source lumineuse (2).

23. Dispositif selon la revendication 22, **caractérisé en ce que** le filtre (23) est un élément de filtre diélectrique.
